# EUROPEAN PATENT SPECIFICATION

(11) **EP 3 013 297 B1**
(45) Date of publication and mention of the grant of the patent: **23.08.2017**
(21) Application number: 14742410.5
(22) Date of filing: 26.06.2014
(51) Int. Cl.: A61F 13/20, A61F 13/34, B26D 5/00

(54) **METHOD OF CUTTING A THREAD BETWEEN TWO SUBSTRATES**
VERFAHREN ZUM SCHNEIDEN EINES FADENS ZWISCHEN ZWEI GEGENSTÄNDEN
PROCÉDÉ POUR COUPER UNE CORDELETTE ENTRE DEUX SUBSTRATS

(30) Priority: 27.06.2013 US 201361840178 P
(43) Date of publication of application: 04.05.2016
(73) Proprietor: The Procter & Gamble Company, Cincinnati, OH 45202 (US)
(72) Inventor: DURLING, Evan Joseph, Cincinnati, Ohio 45202 (US); STRONG, Kevin Charles, Cincinnati, Ohio 45202 (US); ORNDORFF, Jason Matthew, Cincinnati, Ohio 45202 (US)
(74) Representative: Kremer, Véronique Marie Joséphine
(86) International application number: PCT/US2014/044215
(87) International publication number: WO 2014/210239

(56) References cited:
- EP-A1- 1 818 033
- DE-U1-202009 005 394
- US-A- 4 133 235
- US-A- 4 355 554
- US-A- 4 771 665
- US-A1- 2008 035 040

## Description

### FIELD OF INVENTION

The present disclosure relates to a method for separating substrates joined by a thread. Specifically, the method relates to the separation of substrates by cutting the thread joining the substrates while using a strain gauge to verify the cut location.

### BACKGROUND OF THE INVENTION

Cords are commonly attached onto tampons to help ease in the removal of the tampon from the vaginal cavity. Typically, during the manufacturing process, a continuous cord is sewn onto multiple pledgets thereby connecting the pledgets. The pledgets may be spaced on a conveyor connected by the continuous cord. The amount of cord between two pledgets may comprise the amount of cord that is not sewn to the pledget, which represents the graspable portion of a withdrawal cord. The cord is then cut between pledgets in a separate process; leaving a portion of the cord attached to the pledget prior to the location of the cut and portion of the cord attached to a pledget after the location of the cut. The typical process also sews the cord onto the entire longitudinal length of the pledget. This leads to unnecessary sewing that utilizes excess sewing thread and cord. This process also creates inefficiency because the conveyor must space the pledgets to account for the desired length of cord between pledgets.

The typical process ideally cuts the cord without cutting any of the pledgets. However, often, one or more pledgets are partially cut when cutting the cord. This leads to irregular pledgets that are discarded because they do not meet the product target.

However, improvements in cord attachment have enabled a discrete cord to be attached to each individual pledget while connecting the pledgets with a thread.

US 2008/035040A1 (Aoyama) discloses a method for producing tampons wherein at least two continuous cords are placed in parallel, the cords being sewn to respective first and second adjacent cotton pads, and including a cord switching step.

US 4,133,235 (Gerber) discloses a method for cutting a sheet material wherein during cutting, a sensor detects a cutting parameter that is affected by the interaction of the cutting blade and sheet material, and signals provided by the sensor are fed back to a closed loop automatic control mechanism to adjust or initiate further steps in the process.

US 4,355,554 (Gregory) discloses an apparatus wherein web sectioning occurs through engagement or interference of the cutting edges of a rotatable knife assembly with the surface of a drum and wherein an indicator is provided for generating a signal indicative of the force resulting from such interference.

US 4,771,665 (Van Doorn) discloses a blade quality monitor for use with cutter reels having specifically identifiable blades utilizing a sensor to detect the position of a physical anomaly on the reel. Additional sensors sense the force at the interface between the cutter reel, the material wrapped thereon and the associated pressure roller.

DE 20 2009 005394U1 (Mueller) discloses a floating-cutter with a device for monitoring the cutting force when cutting paper, cardboard or the like, having at least one sensor which is designed as a strain gauge or a piezoelectric element.

Therefore, it would be desirable to provide a method to cut the thread between the pledgets without cutting the pledget or the discrete cord attached to the pledget while running at a manufacturing speed.

### SUMMARY OF THE INVENTION

A method for cutting the thread between two substrates that includes moving two or more substrates toward a cutting apparatus, wherein the substrates are connected by a thread; contacting the cutting apparatus with the thread, wherein the cutting apparatus cuts the thread; and using a sensor to determine any deviation in a cut force over time versus a standard cutting profile determined for the specific thread material.

A method for cutting the thread between two substrates that includes moving two or more substrates toward a cutting apparatus comprising a drum with one or more vacuum ports and a plurality of cutting implements that are equally spaced along the outer perimeter of the drum, wherein the substrates are connected by a thread. The method further includes contacting the cutting apparatus with the thread, wherein the cutting apparatus cuts the thread; and using a sensor to determine any deviation in a cut force over time versus a standard cutting profile determined for the specific thread material.

### BRIEF DESCRIPTION OF THE DRAWINGS

While the specification concludes with claims particularly pointing out and distinctly claiming the subject matter of the present invention, it is believed that the invention can be more readily understood from the following description taken in connection with the accompanying drawings, in which:
FIG. 1 is a perspective view of the apparatus.
FIG. 2 is a perspective view of the apparatus.
FIG. 3 is a cross section view of a cutting apparatus taken along 3-3 of FIG.2.
FIG. 4 is a close up view of a cutting implement taken from FIG. 3.
FIG. 5 exemplifies a pledget in a flat-out, uncompressed state.
FIG. 6 represents a graph showing the cut force over time for two samples.

### DETAILED DESCRIPTION OF THE INVENTION

The following definitions may be useful in understanding the present disclosure.

"Compressed" refers herein to pressing or squeezing together or otherwise manipulating the size, shape, and volume to obtain a generally elongated absorbent member having a vaginally insertable shape.

"Cross direction" (CD) refers herein to a direction that is not parallel with, and usually perpendicular to, the machine direction.

"Fluid flow" refers herein to the flow of a medium. The path taken by the medium defines a fluid flow path.

"Machine direction" (MD) refers herein to the direction of material flow through a process. In addition, relative placement and movement of material can be described as flowing in the machine direction through a process from upstream in the process to downstream in the process.

The terms "pledget" and "tampon pledget" refer herein to a construction of absorbent material prior to the compression of such construction into a tampon as described below. A pledget may be in the form of a chevron.

Tampon pledgets are sometimes referred to as tampon "blanks" or "softwinds," and the term "pledget" is intended to include such terms as well.

As used herein, a "substrate" relates to a material or a combination of materials that create a first plane and a second plane, opposite the first plane in any three dimensional shape. The substrate may be in the form of a sheet, such as, for example, a pledget, a plate, a sheet of glass, and a sheet of material. The substrate can comprise, for example, cellulose based materials, fibrous materials, metals, glass, silicate materials, thermoplastics, and thermoset plastics. The substrate can be paper, paperboard, cardboard, cellulose, such as, e.g., molded cellulose, or any combinations thereof, polypropylene, polybutylene, polystyrene, polyvinylchloride, polyacrylate, polymethacrylate, polyacrylonitrile, polyacrylamide, polyamide, nylon, polyimide, polyester, polycarbonate, polylactic acid, poly hydroxyalkanoate, ethylene vinyl acetate, polyurethane, silicone, derivatives thereof, copolymers thereof, mixtures thereof, or any plastic material.

The substrate can be non-absorbent or absorbent and can include any suitable material, such as, for example, a fibrous nonwoven material comprising natural, synthetic, or a blend of natural and synthetic fibers. Suitable synthetic fibers can include, e.g., fibers such as polyester, polyolefin, nylon, polypropylene, polyethylene, polyacrylic, cellulose acetate, polyhydroxyalkanoates, aliphatic ester polycondensates, bicomponent fibers and/or mixtures thereof. Natural fibers can include, e.g., rayon and those commonly known to be non-synthetic and of natural origin such as cotton. The fibers can have any suitable cross-sectional shape, such as, e.g., round, tri-lobal, multi-lobal, delta, hollow, ribbon-shaped, and/or any other suitable shape, or mixtures thereof. Fibers with any suitable diameter can be used, such as, e.g., from about 0.5 to about 50 microns, such as, e.g., from about 1 to about 30 microns, such as, e.g., from about 10 to about 25 microns. Fiber diameter can be determined using any suitable means; however, for non-round fibers, diameter can typically be determined by reference to the diameter of a fiber with the same cross-sectional area as the non-round fiber.

The term "tampon," as used herein, refers to any type of absorbent member that is inserted into the vaginal cavity or other body cavities for the absorption of fluid therefrom. Typically, tampons are constructed from a generally elongated absorbent member that has been compressed or formed into a vaginally insertable shape.

As used herein, the term "thread" refers to a pliable strand, cord, or filament of natural or manufactured material.

The term "vaginal cavity" refers herein to the internal genitalia of the human female in the pudendal region of the body. The term "vaginal cavity" as used herein is intended to refer to the space located between the introitus of the vagina (sometimes referred to as the sphincter of the vagina) and the cervix and is not intended to include the interlabial space, including the floor of the vestibule. The external visible genitalia generally are not included within the term "vaginal cavity" as used herein.

The term "volume" refers herein to the volume of the fibers and the void space within the pledget. Volume is measured by the multiplication of the length by the width by the thickness of the pledget.

The present disclosure relates to a method for cutting a thread between two substrates. The method includes moving two or more substrates toward a cutting apparatus, wherein the substrates are connected by a thread. A discrete cord may be attached to each substrate. The method further includes cutting the thread using a cutting implement while monitoring the cut force versus time relationship to create a cut force profile for each cut. The method further includes comparing the cut force profile calculated with prior cut force data. The cut force may then be utilized to determine if any portion beyond the thread is cut.

In an exemplary configuration, the method may further include moving the discrete cords via vacuum. Moving the discrete cords may include drawing a portion of the discrete cords into a vacuum port.

The defect substrates may be marked for disposal after cutting the thread. The defect substrates may be marked electronically for ejection at any suitable point in the process.

The cutting apparatus comprises a cutting implement enabled to sever the thread between two substrates, such as, for example, a rigid knife, a rotary knife, a flexible knife, a guillotine, or a blade. In an exemplary configuration, a knife is attached to a rotary axis that rotates the knife through the substrate path. A pressure surface, such as, for example, a rotating anvil, may be located opposite the knife. The knife may contact the pressure surface, severing the thread between substrates as they pass by the cutting apparatus. The cutting apparatus may be configured to cut the thread between about 150 to about 1500 times per minute.

The cutting apparatus may comprise a drum with multiple, equally spaced cutting implements. In a non-limiting embodiment, the drum may comprise vacuum ports enabled to draw in the discrete cords.

The cutting apparatus may comprise one or more vacuum ports. The vacuum ports may draw in any loose attachments to the substrate. This moves the loose attachments away from the cutting implement. Loose attachments may include, for example, a withdrawal cord attached to the substrate.

A sensor derives the cut force over time to create a cut force profile for each cut. The sensor may be, for example, a strain gauge. The strain gauge may be any suitable strain gauge enabled to calculate the cutting force. The strain gauge may be a semiconductor strain gauge, a foil gauge, a mercury-in-rubber strain gauge, a vibrating wire strain gauge, or a capacitive strain gauge.

The strain gauge may be mounted on the cutting apparatus blade, a knife holder, a knife anvil, a suitable location that can monitor cut force, or combinations thereof. The cutting apparatus may comprise more than one strain gauge. The strain gauge may measure the deflection in the blade and convert the information to determine a cut force profile. The cut force profile may be compared between each pair of substrates to identify rejects.
In a non-limiting embodiment, the sensor may be a laser. The laser may measure deflection of one or more components of the cutting apparatus to derive the cut force profile.

The thread joining the substrates may comprise any suitable pliable material, including for example, aluminum, copper, gold, silver, steel, cotton, cellulose, rayon, polyolefins such as, for example, polyethylene or polypropylene, nylon, silk, polytetrafluoroethylene, wax, Teflon, or any other suitable materials.

The thread may be non-absorbent along at least the location of attachment to the pledget. As used herein, the term "non-absorbent" refers to a structure that does not retain a significant portion of deposited fluid in its structure. The entire thread may be made non-absorbent, if desired. The materials comprising the cord may be inherently non-wettable or hydrophobic, or they may be treated to provide such properties. For example, a coating of wax may be applied to the cord to decrease or eliminate its absorbency. The thread need not necessarily be non-wicking, even if a non-absorbent thread is desired.

The substrates may each have a discrete cord. The discrete cord may alternate between a distinguishing feature and a length of plain cord. One discrete cord may be made up of a distinguishing feature and one or more lengths of plain cord. The distinguishing feature may be a slub. The slub may be attached onto the pledget. The length of plain cord may extend beyond the pledget. The length of the slub and the length of the plain cord are predetermined in the cord supply. The cord supply may comprise a continuous cord that alternates between slubs and non-slub portions or lengths of plain cord.

The discrete cord may be any suitable length, such as, for example, between 10 mm and 200 mm, between 20 mm and 150 mm, between 20 mm and 100 mm, 200 mm or less, 150 mm or less, 100 mm or less, such as for example, 90 mm, 80 mm, 70 mm, 60 mm, 50 mm, 40 mm, 30 mm, 20 mm, 10 mm, 9 mm, 8 mm, 7 mm, 6 mm, 5 mm, 4 mm, 3 mm, 2 mm, or 1 mm. The slub may be any suitable length, such as, for example, 100 mm or less, such as for example, 50 mm, 45 mm, 40 mm, 35 mm, 30 mm, 25 mm, 20 mm, 15 mm, 10 mm, 5 mm, 4 mm, 3 mm, 2 mm, or 1 mm. The slub may be a percentage of the total discrete cord length, such as, for example, 50%, 45%, 40%, 35%, 30%, 25%, 20%, 15%, 10%, or 5%.

A vacuum manifold may draw the discrete cord away as it moves down the conveyor. Alternatively, the discrete cord may be held by friction by two or more tangible bodies that run along the conveyor track.

The substrate may be a pledget. The pledget may comprise rayon, cotton, or combinations of both materials. These materials have a proven record of suitability for use in the human body. The rayon used in the absorbent material may be any suitable type typically used in disposable absorbent articles intended for in vivo use. Such acceptable types of rayon include GALAXY Rayon (a tri-lobed rayon structure) available as 6140 Rayon from Acordis Fibers Ltd., of Hollywall, England. SARILLE L Rayon (a round fiber rayon), also available from Acordis Fibers Ltd. is also suitable. Any suitable cotton material may be used in the absorbent material. Suitable cotton materials include, long fiber cotton, short fiber cotton, cotton linters, T-fiber cotton, card strips, and comber cotton. The cotton may be scoured and bleached cotton absorbent with a glycerin finish, or other suitable finish.

The pledget may comprise a first end, middle section, and a second end along a longitudinal axis. The first end may also correspond to the withdrawal end to which a withdrawal cord may be attached. The second end may also correspond to the insertion end. The pledget may comprise absorbent layers comprising absorbent fiber materials.

The pledget may be any suitable shape, size, material, or construction for compression or formation into a tampon having a vaginally insertable shape. The pledget may be generally square or rectangular or take on other shapes such as trapezoidal, triangular, hemispherical, chevron or hourglass shapes.

The pledget may be a laminar structure comprised of integral or discrete layers. The absorbent material may comprise 100% rayon fibers or 100% cotton fibers. The absorbent material may comprise a combination of rayon and cotton fibers in any suitable combination. The absorbent material may comprise greater than about 25%, 30% or 40% rayon fibers and the balance of the absorbent material comprising cotton fibers. The absorbent material may comprise greater than about 50% rayon fibers with cotton fibers comprising the balance of the absorbent material. The absorbent material may comprise greater than about 60, 70, 75, 80 or 90 % rayon fibers and the balance of the absorbent material comprising cotton fibers. In one layered configuration, each of the layers may comprise essentially 100% of the same material, such as outer layers of 100% rayon and an intermediate layer of 100% cotton. A Super Plus absorbency tampon may be made from a pledget comprising about 100% rayon fibers. A Super absorbency or Regular absorbency tampon may be made from a pledget comprising about 25% cotton and about 75% rayon fibers. A Junior absorbency tampon may be made from a pledget comprising about 50% cotton and about 50% rayon fibers.

The pledget may be constructed from a wide variety of liquid-absorbing materials commonly used in absorbent articles such as rayon (including tri-lobal and conventional rayon fibers), cotton, or comminuted wood pulp which is generally referred to as airfelt. Examples of other suitable absorbent materials include, but are not limited to, creped cellulose wadding; meltblown polymers including coform; chemically stiffened, modified or cross-linked cellulosic fibers; synthetic fibers such as crimped polyester fibers; peat moss; foam; tissue including tissue wraps and tissue laminates; or any equivalent material or combinations of materials, or mixtures thereof.

Typical absorbent materials may comprise cotton, rayon folded tissues, woven materials, nonwoven webs, synthetic and natural fibers, or sheeting. The pledget and any component thereof, may comprise a single material or a combination of materials. Additionally, superabsorbent materials, such as superabsorbent polymers or absorbent gelling and open-celled foam materials, may be incorporated into the tampon.

Examples of the absorbent fiber materials used for the absorbing layer include hydrophilic fibers such as cotton, rayon and synthetic fiber. Single or multiple fiber webs, nonwoven or woven fabrics, preferably having a weight of 150 g/m² to 1,500 g/m² and a thickness of substantially 0.1 mm to 0.9 mm are lapped over another absorbent fiber material to form an absorbing layer having a thickness of 1.0 mm to 15 mm and preferably having a thickness of 2.0 mm to 10 mm are used as the absorbing layer.

Fiber webs and nonwoven fabrics may be shaped by card webbing, air-laying method, wet laid method and the like, on a base such as a synthetic fiber sheet. Hydrophobic fibers or hydrophobic fibers comprising a hydrophilic property may also be comprised in the absorbing layer with the hydrophilic fibers. In addition, compounds having a water absorbing property, such as polymers with a high water absorbing property, may be comprised in the absorbing layer. The surface material with liquid permeability is made of nonwoven fabrics formed by hydrophobic fibers or mesh films, to which mesh treatment has been performed. The type of nonwoven fabrics used for the surface material is not particularly limited and examples include spunbond nonwoven fabrics, spunlace nonwoven fabrics and thermal bond nonwoven fabrics.

The hydrophobic fiber which makes up the nonwoven fabrics is not particularly limited and examples include fibers of polyester, polypropylene and polyethylene. The weight of the nonwoven fabrics is between 8 g/m² to 40 g/m².

A typical size for the pledget prior to compression may be from about 30 or 40 mm to about 60, 70, 80, 90 or 100 mm in length and from about 40 or 50 mm to about 70, 75, 80, 85, or 90 mm in width. The typical range for the overall basis weight may be from about 150, 200, or 250 gsm to about 600, 800, 1000 or 1100 gsm.

In a non-limiting configuration, a pledget may advance in a machine direction along a conveyor track to the sewing apparatus that engages the discrete cord. The pledget may be oriented such that the first end is perpendicular with the machine direction. In such a non-limiting configuration, the discrete cord sewing apparatus may be configured to sew a portion of a single discrete cord to the pledget utilizing thread. The thread will connect the pledget to the next pledget in a chain. The sewn portion of the single discrete cord may be sewn along the longitudinal axis of the pledget in the first end of the pledget. Alternatively, the sewn portion of the single discrete cord may be sewn along the longitudinal axis of the pledget in the middle section. The single discrete cord extends beyond the pledget. The discrete cord may be sewn onto the pledget by one or more stitches, such as, for example, between 1 and 20 stitches, such as, for example, 2 stitches, 3 stitches, 4 stitches, 5 stitches, 6 stitches, 7 stitches, 8 stitches, 9 stitches, or 10 stitches.

The conveyor may deliver the substrates to the cutting apparatus such that the cutting apparatus cutting implement cuts the thread between two substrates. The conveyor and the cutting apparatus may be configured such that the cutting implement crosses the substrate path once per substrate; separating the substrate from the chain of substrates on the conveyor. The substrate may then be rotated on a drum.

A sensor derives a cut force profile over time. The data is compared to a predetermined standard. The standard may be specific for the properties of the thread, such as, for example, material, thickness, and elasticity of the thread. It may be understood by one of ordinary skill in the art that a new standard may be developed for each possible type of thread intended to be cut by the cutting apparatus.

The conveyer and the infeed may be adjusted to space the pledgets apart based on the discrete cord setting. The conveyer may feed the pledget to a sewing apparatus. The conveyor may be timed so that the number of pledgets is timed with the number of discrete cords to have one pledget for every one discrete cord.

While the present disclosure discusses an apparatus for delivering a discrete cord to a pledget, it is to be appreciated that the methods and apparatuses disclosed herein may be used to deliver a cord to any form of substrate that has a discrete cord attached onto the substrate.

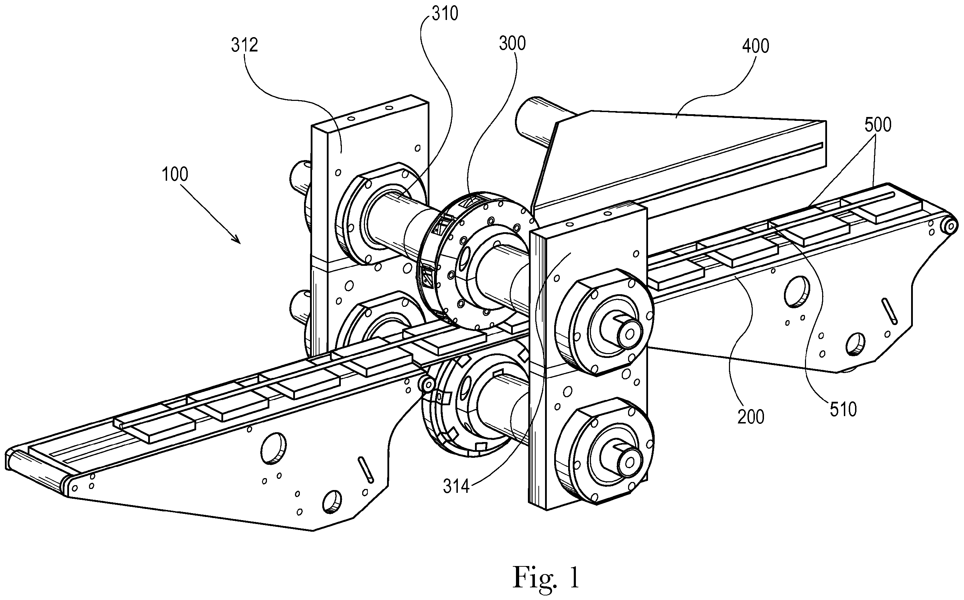

FIG. 1 shows a simplified flowchart of the apparatus 100. The apparatus 100 comprises a conveyor 200, and a cutting apparatus 300. The apparatus may optionally comprise a vacuum manifold 400 that is parallel to the conveyor 200. A series of substrates 500 connected by a thread 510 are located on the conveyor prior to the cutting apparatus 300. The cutting apparatus 300 separates the substrates 500 by cutting the thread 510 between each pair of substrates 500. The cutting apparatus 300 may be on a rotary axis 310 between two plates 312,314 that hold the cutting apparatus 300 in place. The rotary axis 310 is connected to a driving mechanism.

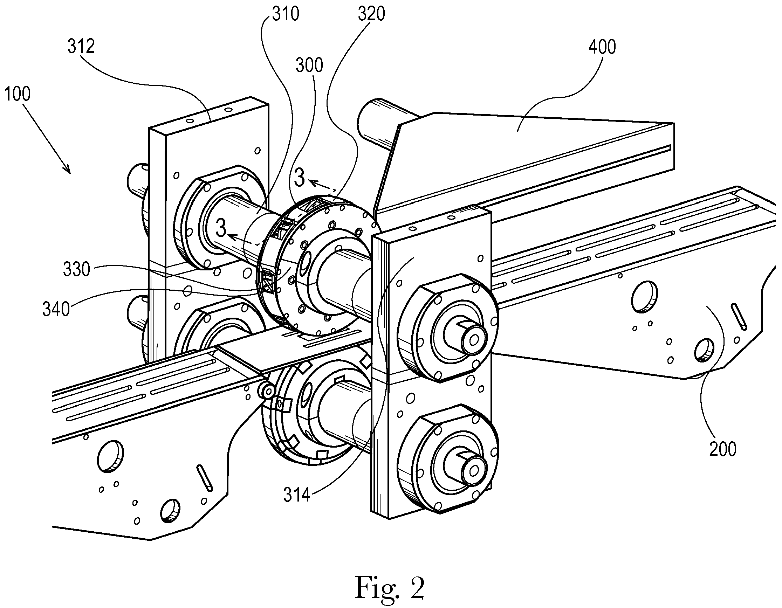

FIG. 2 shows an exemplary configuration of the apparatus 100. The apparatus 100 has a conveyor 200, a vacuum manifold 400, and a cutting apparatus 300 comprising equally spaced cutting implements 320 on a drum 330. The drum is on a rotary axis 310 between two plates 312,314 that hold the cutting apparatus 300 in place. The cutting apparatus 300 has a vacuum source inside the drum 330 and vacuum ports 340 on the exterior of the drum between cutting implements 320.

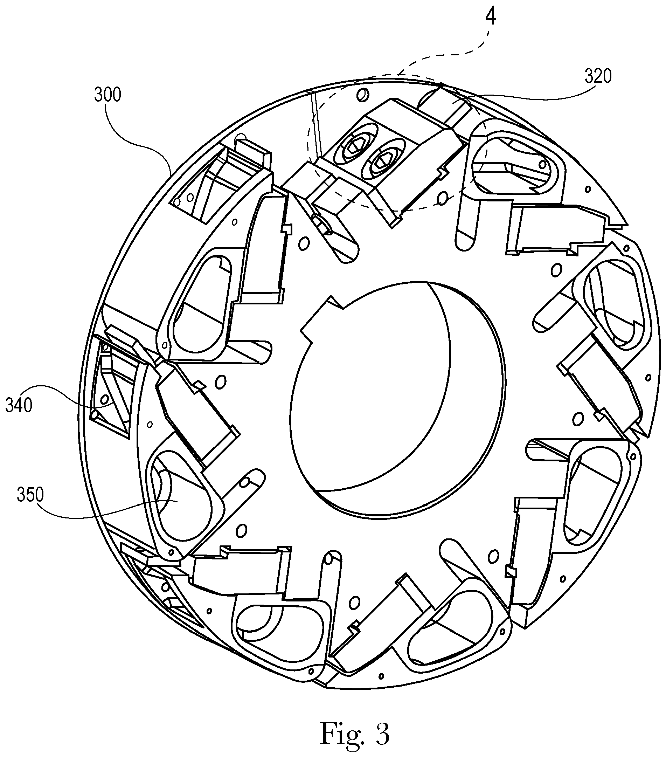

FIG. 3 shows a cross section of the cutting apparatus 300 taken along 3-3 of FIG. 2. The cutting apparatus 300 has multiple cutting implements 320 equally spaced along the cutting apparatus 300. The cutting apparatus 300 also has vacuum ports 340 along the outer surface of the cutting apparatus 300. The vacuum ports connect to the inside of the drum to a vacuum opening 350

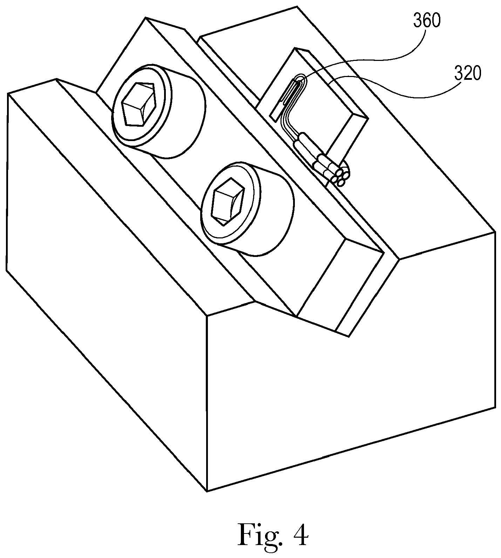

FIG. 4 shows a close up of the cutting implement 320 taken from FIG. 3. As shown in FIG. 4, a strain gauge 360 may be located on the cutting implement 320.

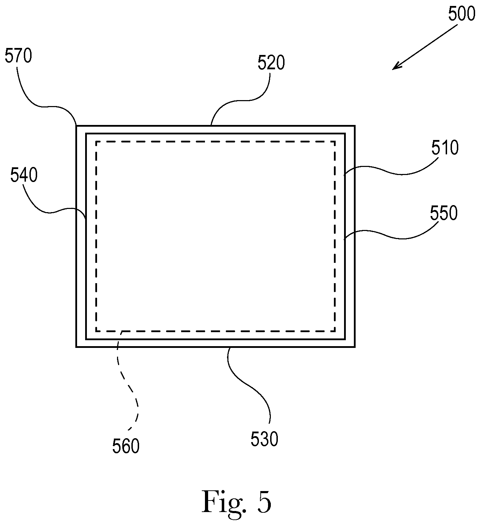

FIG. 5 exemplifies a tampon pledget 500 in a flat-out, uncompressed state. The absorbent material 510 may form a tampon pledget 500. The tampon pledget 500 comprises an insertion end 520, a withdrawal end 530, a first longitudinal edge 540, and a second longitudinal edge 550. Compression of a tampon pledget 500 can form a compressed absorbent member. The pledget 500 can have an overwrap 560 and an additional optional second overwrap 570.

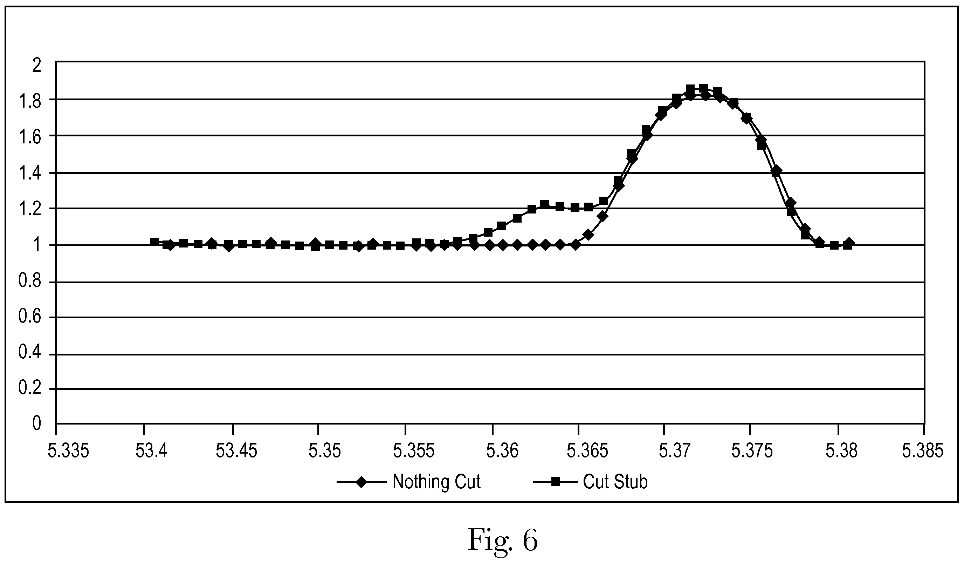

FIG. 6 exemplifies a graphical representation of the cut force versus time. The graph shows a curve wherein the only item cut is the thread versus a curve wherein the cutting implement also contacts a portion of the substrate or a withdrawal cord in addition to the thread. The line labeled "Nothing cut" represents wherein the only item cut is the thread and the line labeled "Cut slub" represents wherein the cutting implement also contacts a portion of the substrate or a withdrawal cord in addition to the thread.

The citation of any document is not an admission that it is prior art with respect to any invention disclosed or claimed herein or that it alone, or in any combination with any other reference or references, teaches, suggests or discloses any such invention. Further, to the extent that any meaning or definition of a term in this document conflicts with any meaning or definition of the same term in a documentreferenced, the meaning or definition assigned to that term in this document shall govern.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of this invention.

## Claims

1. A method for cutting the thread between two substrates, the method comprising:
moving two or more substrates toward a cutting apparatus, wherein the substrates are connected by a thread;
contacting the cutting apparatus with the thread, wherein the cutting apparatus cuts the thread; and
using a sensor to determine any deviation in a cut force over time versus a standard cutting profile determined for the specific thread material;
wherein the method further comprises marking a substrate for reject when a cut force exceeding the nominal baseline level occurs prior to normal thread cut deflection.

2. The method of claim 1, wherein the cutting apparatus is configured to cut the thread between about 150 to about 1500 times per minute.

3. The method of any preceding claim, wherein the cutting apparatus contacts an anvil when cutting through the thread.

4. The method of any preceding claim, wherein one or more sensors are mounted on the cutting apparatus blade, a knife holder, a knife anvil, a suitable location that can monitor cut force, or combinations thereof.

5. The method of any preceding claim, wherein the substrate is a pledget.

6. The method of any preceding claim, wherein comparing the cut force profile calculated by the strain gauge with prior cut force data uses a database of cut force data which is particular to a thread material.

7. The method of any preceding claim, wherein monitoring the cut position further includes monitoring the knife rotational position.

8. The method of any preceding claim, wherein the cutting apparatus is a flex knife.

9. The method of any preceding claim, wherein the method further comprises moving the discrete cord by vacuum.

10. The method of any preceding claim, wherein the cutting apparatus comprises a drum with one or more vacuum ports and a plurality of cutting implements that are equally spaced along the outer perimeter of the drum.

## Patentansprüche

1. Verfahren zum Schneiden des Fadens zwischen zwei Substraten, wobei das Verfahren umfasst:
Bewegen zweier oder mehrerer Substrate in Richtung einer Schneidvorrichtung, wobei die Substrate durch einen Faden verbunden sind;
Inkontaktbringen der Schneidvorrichtung mit dem Faden, wobei die Schneidvorrichtung den Faden schneidet; und
Verwenden eines Sensors zur Bestimmung von Abweichungen der Schneidkraft über einen Zeitraum gegenüber einem Standardschnittprofil, das für das spezifische Fadenmaterial festgelegt wurde;
wobei das Verfahren ferner das Markieren eines Substrats für den Verwurf umfasst, wenn eine Schneidkraft angewandt wird, die das nominale Ausgangsniveau vor normaler Fadenschneidumlenkung übersteigt.

2. Verfahren nach Anspruch 1, wobei die Schneidvorrichtung so konfiguriert ist, dass der Faden zwischen 150 und 1500 Mal pro Minute geschnitten wird.

3. Verfahren nach einem der vorstehenden Ansprüche, wobei die Schneidvorrichtung beim Schneiden durch den Faden auf einen Amboss trifft.

4. Verfahren nach einem der vorstehenden Ansprüche, wobei ein oder mehrere Sensoren an der Klinge der Schneidvorrichtung angebracht sind, sowie eine Messerhalterung, ein Messeramboss, eine angemessene Position zur Überwachung der Schneidkraft, oder Kombinationen davon.

5. Verfahren nach einem der vorstehenden Ansprüche, wobei das Substrat ein Bausch ist.

6. Verfahren nach einem der vorstehenden Ansprüche, wobei zum Vergleich des vom Belastungsmessgerät errechneten Schneidkraftprofils mit vorhergehenden Schneidkraftdaten eine Datenbank mit Schneidkraftdaten verwendet wird, die typisch für ein Fadenmaterial sind.

7. Verfahren nach einem der vorstehenden Ansprüche, wobei das Überwachen der Abschneidestelle ferner die Überwachung der Messerrotationsposition einschließt.

8. Verfahren nach einem der vorstehenden Ansprüche, wobei die Schneidvorrichtung ein Flex-Messer ist.

9. Verfahren nach einem der vorstehenden Ansprüche, wobei das Verfahren ferner das Bewegen der separaten Schnur mittels eines Vakuums umfasst.

10. Verfahren nach einem der vorstehenden Ansprüche, wobei die Schneidvorrichtung eine Walze mit einem oder mehreren Vakuumanschlüssen und einer Vielzahl von Schneidwerkzeugen umfasst, die in gleichmäßigem Abstand entlang des äußeren Rands der Walze angeordnet sind.

## Revendications

1. Procédé pour couper le fil entre deux substrats, le procédé comprenant les étapes consistant à :
déplacer deux substrats ou davantage vers un appareil de coupe, dans lequel les substrats sont reliés par un fil ;
mettre le fil en contact avec l'appareil de coupe, dans lequel l'appareil de coupe coupe le fil ; et
utiliser un capteur pour déterminer toute déviation dans une force de coupe au fil du temps par rapport à un profil de coupe standard déterminé pour le matériau de fil spécifique,
dans lequel le procédé comprend en outre le marquage d'un substrat pour rejet lorsqu'une force de coupe dépassant le niveau de base de référence nominal se produit avant la déflexion de coupe normale du fil.

2. Procédé selon la revendication 1, dans lequel l'appareil de coupe est configuré pour couper le fil entre 150 fois par minute environ et 1500 fois par minute environ.

3. Procédé selon l'une quelconque des revendications précédentes, dans lequel l'appareil de coupe vient en contact avec une enclume lors du découpage du fil.

4. Procédé selon l'une quelconque des revendications précédentes, dans lequel un ou plusieurs capteurs sont montés sur la lame de l'appareil de coupe, un porte-couteau, une enclume de couteau, un emplacement approprié qui peut surveiller la force de coupe ou des combinaisons de ces éléments.

5. Procédé selon l'une quelconque des revendications précédentes, dans lequel le substrat est une compresse.

6. Procédé selon l'une quelconque des revendications précédentes, dans lequel la comparaison du profil de force de coupe calculé par la jauge de contrainte avec des données de forces de coupe antérieures utilise une base de données de forces de coupe spécifiques à un matériau de fil.

7. Procédé selon l'une quelconque des revendications précédentes, dans lequel la surveillance de la position de coupe comprend en outre la surveillance de la position de rotation du couteau.

8. Procédé selon l'une quelconque des revendications précédentes, dans lequel l'appareil de coupe est un couteau flexible.

9. Procédé selon l'une quelconque des revendications précédentes, dans lequel le procédé comprend en outre le déplacement du cordon discret par aspiration.

10. Procédé selon l'une quelconque des revendications précédentes, dans lequel l'appareil de coupe comprend un tambour doté d'un ou plusieurs orifices d'aspiration et une pluralité d'outils de coupe qui sont également espacés le long du périmètre externe du tambour.
